(19)

Europäisches Patentamt

European Patent Office

Office européen des brevets

(11) **EP 4 656 147 A1**

(12) **EUROPEAN PATENT APPLICATION**

(43) Date of publication:
**03.12.2025   Bulletin 2025/49**

(21) Application number: **24178411.5**

(22) Date of filing: **28.05.2024**

(51) International Patent Classification (IPC):
**A61B 17/64** (2006.01)      **A61B 17/66** (2006.01)

(52) Cooperative Patent Classification (CPC):
**A61B 17/6433; A61B 17/66;** A61B 17/6416;
A61B 17/6458; A61B 2017/00991

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Georg-August-Universität Göttingen Stiftung**
**Öffentlichen Rechts, Universitätsmedizin**
**37075 Göttingen (DE)**

(72) Inventors:
• **Li, Xishan**
  **37075 Göttingen (DE)**

• **Zhou, Xiang**
  **37075 Göttingen (DE)**
• **Böker, Dr. Kai**
  **37085 Göttingen (DE)**
• **Schilling, Arndt**
  **37075 Göttingen (DE)**
• **Lehmann, Wolfgang**
  **37075 Göttingen (DE)**

(74) Representative: **REHBERG HÜPPE + PARTNER**
**Patentanwälte PartG mbB**
**Robert-Gernhardt-Platz 1**
**37073 Göttingen (DE)**

(54) **PELVIC RING INJURY TREATMENT DEVICE AND PELVIC RING INJURY TREATMENT ASSEMBLY**

(57)      The invention relates to a pelvic ring treatment device (19). The pelvic ring injury treatment device (19) comprises a base body (25) and two Schanz pin accommodations (54) for accommodating two Schanz pins (10, 11). The invention proposes that the pelvic ring injury treatment device (19) comprises two fixing devices (32) for fixing the Schanz pins (10, 11) in the Schanz pin accommodations (54). Furthermore, the pelvic ring injury treatment device (19) comprises two rotary links (33) for rotating the Schanz pin accommodations (54) relative to the base body (25) about axes of rotation (20, 21). According to the invention the Schanz pin accommodations (54) can be rotated relative to the base body (25) about the axes of rotation (20, 21) also when the fixing devices (32) are in the fixing state. The invention allows a simultaneous application of compression forces both to the anterior joint and the posterior joint of the pelvic ring.

**Fig. 3**

EP 4 656 147 A1

## Description

### TECHNICAL FIELD OF THE INVENTION

The pelvis is a critical structure in the human skeletal System. The pelvis is formed by the iliac bones, joined anteriorly by an anterior joint to the symphysis pubis and posteriorly by a posterior joint to the sacrum. The pelvis transmits loads from the spine to the lower limbs through the sacroiliac joint, supported by a robust ligamentous complex. Additionally, it serves as the origin for major muscles that are essential for posture and gait. Gaps of a pelvic ring can be due to an injury, in particular by a fracture or a dislocation. Gaps, particularly those involving the rupture or dislocation of the pelvic ring at the anterior joint and/or the posterior joint, pose a significant clinical challenge due to their complexity and the associated hemorrhagic complications. These fractures or dislocations often accompany vascular and nerve damages, systemic coagulation disorders, deep vein thromboses in the lower limbs, and a hemorrhagic shock, posing a critical threat to the patient's survival.

The placement of external pelvic fixators outside the skin to stabilize bone fragments with Schanz pins are a mainstay in emergency treatment protocols due to their simplicity and ease of placement. However, current designs have limitations. They often do not provide simultaneous stabilization of both the anterior joint and the posterior joint, nor do they offer adjustable compression of the gaps. A lack of dual stabilization and compression of current designs is particularly problematic in unstable fractures of the pelvis involving both the anterior and posterior joint, which can lead to high rates of disability and mortality.

### PRIOR ART

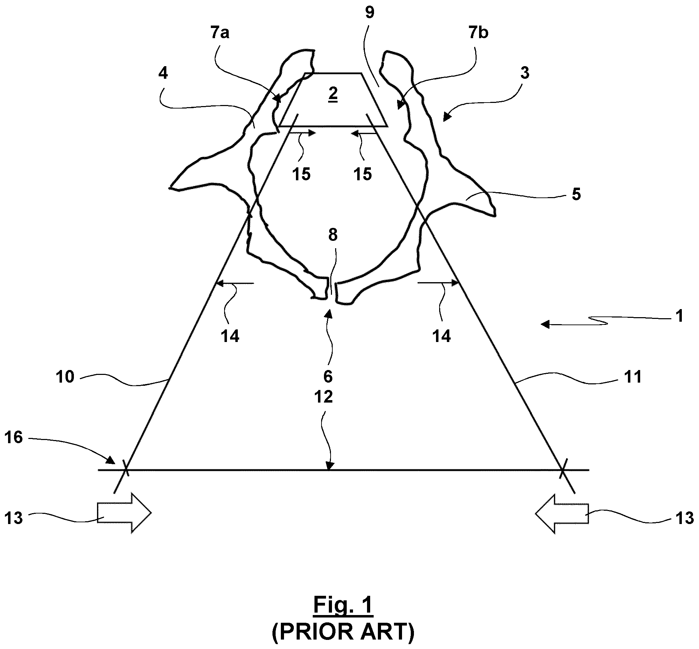

**Fig. 1** is a very schematic view of a prior art pelvic ring injury treatment assembly 1. In Fig. 1 the sacrum 2 is schematically represented by a trapezium. The pelvic ring 3 further comprises the innominate bones 4, 5 directly linked to each other by the anterior joint 6 and linked to the sacrum 2 by posterior joints 7a, 7b. The schematic Fig. 1 shows a gap 8 at the anterior joint 6 as well as a gap 9 at the posterior joint 7b. For the treatment Schanz pins 10, 11 are driven into the innominate bones 4, 5. By a biasing device 12 the free ends of the Schanz pins 11 are biased by biasing forces 13, here a compressing force. Once the desired biasing force 13 has been applied, the Schanz pins 10, 11 are fixed to the biasing device 12 for upholding the biasing force 13 and for maintaining the compression during the healing process. However, the mechanical free body diagram of the Schanz pins 10, 11 shows that for a static equilibrium of the forces and moments acting on the Schanz pins 10, 11 the support by the anterior joint 6 forms a kind of swivel joint for the Schanz pins 10, 11 with the danger that the ends of the innominate bones 4, 5 at the posterior joints 7a, 7b are pressed in outward direction so that the gap 9 is not closed by a compression but opened which is undesired.

The Chinese patent CN 111 529 029 A discloses a pelvic ring injury treatment assembly comprising a closed complex and bulky frame. The frame serves for holding the end regions of two strut branches comprising pivotably linked branch parts and being connected to each other at the free ends at the pelvic ring by a connecting screw. The two branches have to be fixed at a plurality of fixture points to the pelvic ring. The pelvic ring injury treatment assembly has a number of degree of freedom requiring a complex adjustment to the anatomy of the patient.

The European patent application EP 2 837 346 A1 (cp. also the publication "Simultaneous anterior and posterior compression of the pelvic ring with external fixation using a pre-tensed curved bar: A biomechanical study", A. Queipo-de-Llano, A. Perez-Blanca, F. Ezquerro, F. Luna-González in Injury, Int. J. Care Injured 44 (2013) 1787-1792) discloses a pelvic ring injury treatment assembly comprising an frame on which an elastic bar is supported and biased into a C-shape having a specific curvature. When two Schanz pins have been driven into the pelvic bone of the patient the free ends of the Schanz pins are connected to the elastic bar by ball and socket joints and then fixed to the elastic bar. The frame then releases the elastic bar so that the elastic bias of the elastic bar tending to reduce the curvature of the C-shape applies a force and moment on the Schanz pins. The Schanz pins transfer this force and moment to the bone fragments with the aim to reposition the bone fragments. If the initial curvature of the elastic bar has been dimensioned correctly, the bone fragments are pressed against one another with the desired force.

DE 10 2010 032 465 A1 discloses a pelvic ring injury treatment assembly comprising two crossbar control mechanisms each comprising two crossbars linked for being pivoted to each other similar to scissors. The crossbars of each crossbar control mechanism are connected at one end region to an associated Schanz pin. One crossbar control mechanism is connected to the Schanz pins at a smaller distance from the pelvic ring than the other crossbar control mechanism. A separate biasing by suitable biasing devices is required for both crossbar control mechanisms which requires a cumbersome adjustment process. Furthermore, the two crossbar control mechanisms lead to a complex and bulky design.

### OBJECT OF THE INVENTION

It is the object of the present invention to provide a pelvic ring injury treatment device and a pelvic ring injury treatment assembly which is in particular improved with respect to

- the forces, moments, force ratios and/or moment ratios applied by the Schanz pins to the anterior and posterior joint of the pelvic ring and/or
- the complexity of the design and/or
- the production costs and/or
- the time required for the application and/or
- the comfort for the patient and/or
- the reduction of the risk of secondary injuries and complications and/or
- the weight and/or
- the use for a minimally invasive procedure and/or
- the reduction of the required skills of the surgeon when applying and biasing the pelvic ring injury treatment device.

## SOLUTION

According to the present invention, the object of the invention is solved by the features of the independent claims. Additional preferred embodiments according to the invention are to be seen in the dependent claims.

## DESCRIPTION OF THE INVENTION

The invention proposes a pelvic ring injury treatment device which comprises a base body and two Schanz pin accommodations for accommodating two Schanz pins. The inventive pelvic ring injury treatment device comprises two fixing devices for fixing the Schanz pins in the associated Schanz pin accommodations. Furthermore, the inventive pelvic ring injury treatment device comprises two rotary links which allow a rotation of the Schanz pin accommodations (and of the Schanz pins fixed therein by the fixing devices) relative to the base body about axes of rotation defined by the rotary links.

The invention proposes that the fixing devices are independent from the rotary links such that the Schanz pin accommodations can be rotated relative to the base body about the axes of rotation when the fixing devices are in the fixing state.

One embodiment of the pelvic ring injury treatment device is in the following described for explaining the inventive benefit of the new pelvic ring injury treatment device (without restricting the invention to this exemplary embodiment and to the described benefit): According to the invention the Schanz pins might comprise a biasing section wherein a biasing force can be applied by a biasing device to the Schanz pins. Furthermore, the Schanz pins comprise a body section which can be driven into the pelvic ring, in particular the innominate bones. An accommodation section of the Schanz pins is arranged between the biasing section and the body section. In the accommodation section the Schanz pins are fixed by the fixing devices to the base body of the pelvic ring injury treatment device. Due to the remaining rotational degree of freedom about the axes of rotation provided by the two rotary links it is possible to pivot the Schanz pins relative to the base body of the pelvic ring injury treatment device. Compared to the embodiment described under referral to Fig. 1 the inventive pelvic ring injury treatment device and the base body of the same provide an additional connection and articulated support between the two Schanz pins. The force applied by the pelvic ring injury treatment device and the base body to the Schanz pins changes the mechanical free body diagram of the Schanz pins and lead to the result that it is possible to create anterior joint forces 14 and posterior joint forces 15 having the same directions. Depending on the position along the longitudinal axis of the Schanz pins where the Schanz pins are accommodated and fixed to the pelvic ring injury treatment device, it is also possible to dimension the ratio between the anterior joint forces 14 and the posterior joint forces 15 for adapting these forces to the specific fractures or dislocations and to the anatomy of the patient. When the biasing device biases the biasing section of the Schanz pins, the rotary links are effective such that the angle of the Schanz pins relative to each other can change whereas the distance of the Schanz pins at the Schanz pin accommodations does not change. The pivoting movement of the Schanz pins might be used for creating different closing displacements of the gaps 8, 9, the difference in the closure displacements depending on the distance of the gaps 8, 9 from the Schanz pin accommodations.

Within the frame of the invention, the fixing devices for fixing the Schanz pins in the Schanz pin accommodations might have any design. Accordingly, the fixing devices might base on a clamping of the Schanz pins, on a fixing of the Schanz pins by friction and/or by a form lock, by a positive engagement and the like. Here, it is possible that the fixing devices fix the Schanz pins at any limited number of specific and distinct axial positions. However, preferably it is possible to fix the Schanz pins by the fixing devices to the Schanz pin accommodations continuously at least along an axial subsection of the Schanz pins.

For the design of the rotary links any type of rotary link, in particular any rotary bearing or joint, can be used. Preferably, the fixing devices on the one hand side and the rotary links on the other hand side are arranged separately and/or remote from each other or with an offset. Preferably, the fixing device (in particular a clamping ring of the fixing device) and the rotary link (in particular a connection body, a bearing bolt or an axis of rotation of the rotary link) have a distance from each other in a direction perpendicular from a plane spanned by the Schanz pins when driven into the pelvic ring.

It is possible to adjust the anterior joint force and the posterior joint force by changing the biasing force applied by the biasing device. As explained above, the anterior joint force and the posterior joint force and the ratio of the same can also be changed by changing the position where the Schanz pins are accommodated and fixed by the fixing device in the Schanz pin accommodations. For another proposal of the invention, a further adjustment of the anterior joint force and the posterior joint force and of the relative orientation of the Schanz pins can be provided when using a base body which has an adjustable length. Alternatively or cumulatively, the adjustable length can be used for providing a fitting degree of freedom when the Schanz pins have already been driven into the pelvic ring and the pelvic ring injury treatment device has to be coupled to the Schanz pins via the Schanz pin accommodations. Furthermore, the adjustable length of the base body might also be beneficial when during the use of the pelvic ring injury treatment device the fixing positions where the Schanz pins are fixed to the Schanz pin accommodations have to be changed.

Within the frame of the invention, the length of the base body can be adjustable in steps or continuously. Any type of adjustable base body can be used. The base body might e.g. consist of two base body parts which can be mounted or screwed to each other in different relative positions. It is also possible that two base body parts can be secured to each other in continuously adjustable positions by using a friction connection or a clamping connection.

For a particular proposal of the invention, the base body is a telescopic bar. Here, the telescopic bar might comprise a first bar element and a second bar element. In this case, the first bar element comprises a recess. The second bar is arranged in the recess. The telescopic bar is telescoped by creating a relative sliding motion of the second bar element in the recess of the first bar element. The telescoped relative position of the first and second bar elements can then be fixed by a clamping device, a frictional device, a securing device or by a screwed connection. During the telescoping movement the second bar element is guided by the limiting surfaces of the recess. The recess might be a bore having a circular cross section, whereas in this case the second bar element has a circular cross section arranged in the bore. However, preferably the recess and the second bar element have a non-circular circular cross section but e.g. a square or rectangular cross section. By use of non-circular cross sections it is possible to provide a guidance between the bar elements blocking a relative rotational movement about the longitudinal and telescope axis.

In the case that the bar elements have contact surfaces with a circular cross section, a fixation against a rotation about the longitudinal axis can be achieved when the telescoped relative position is fixed e. g. by fixing bolts or fixing a screws accommodated in aligned transverse bores of the bar elements.

The invention also covers embodiments wherein the telescopic bar is a kind of hydraulic cylinder, pneumatic cylinder, electric linear drive and the like, the bar elements in this case being telescoped by the pneumatic, hydraulic or electric bias.

In one particular inventive pelvic ring injury treatment device the first bar element and the second bar element comprise transverse bores which are used for accommodating a fixing bolt or fixing screw. The fixing bolt or fixing screw fixes one of a plurality of relative positions of the bar elements. For adjusting the length of the telescopic bar the fixing bolt or fixing screw is removed from the transverse bores, the length of the telescopic bar is adjusted and then the fixing bolt or fixing screw is inserted into other aligned transverse bores of the bar elements.

Within the frame of the invention there are a lot of options for the design of the rotary links. In one embodiment of the invention, in at least one end region (preferably in both end regions) the base body comprises two parallel bearing walls. The bearing walls each directly or indirectly (e.g. by interposition of a bearing of any type) support a bearing bolt (including a bearing screw) or one common bearing bolt is supported by the two parallel bearing walls. The bearing bolt is part of the associated rotary link and defines the axis of rotation of the Schanz pin accommodations.

It is possible that the bearing bolt is fixed to the parallel bearing walls. In this case, the rotational degree of freedom of the rotary link is provided by supporting an accommodation body for accommodating the Schanz pins in a rotatable way on the bearing bolt. The invention preferably suggests that the bearing bolt is supported by rotational bearings in the parallel bearing walls. In this case, it is possible to fix the at least one bearing bolt to the accommodation body. The rotational bearings provide the desired rotational degree of freedom for allowing a rotation of accommodation body (with the Schanz pins accommodated therein) relative to the base body of the pelvic ring injury treatment device.

As mentioned above there are a lot of options for fixing the Schanz pin accommodation to the Schanz pins. For mentioning only one non-limiting example, it is possible that an accommodation body comprises a bore through which the Schanz pin extends. A setscrew can then be screwed in radial direction into the accommodation body until a front face of the setscrew is pressed against the outer circumference of the Schanz pins for providing the fixation. In a different embodiment of the invention, the Schanz pin accommodations comprise accommodation bodies having the shape of a clamping ring. The clamping ring comprises a slit and is designed and configured for clamping a Schanz pin. Here, the clamping ring might have a bore with an interior diameter being smaller than the diameter of the Schanz pin so that the clamping ring is widened when inserting the Schanz pin and secured to the Schanz pin by the friction induced by its own elasticity. It is also possible that the diameter of the Schanz pins corresponds to the inner diameter of the clamping ring (or there is some gap or play in between). Preferably, a screw connects the end portions of the clamping ring adjacent to the slit, the screw being oriented perpendicular to the front faces of the clamping ring limiting the slit and/or being oriented tangentially to the circumferential direction of the clamping ring. Screwing the screw to the accommodation body leads to a closure of the slit with a reduction of the inner diameter of the bore of the clamping ring. Accordingly, the screwing of the screw creates and adjusts a clamping force by which the clamping ring clamps the Schanz pin.

For another proposal of the invention a connection body comprises at least one bore for accommodating one bearing bolt or a couple of bearing bolts associated with the rotary link. In the case that this rotary link is provided additional to a fixing device comprising a clamping ring with a slit as explained before, the invention optionally proposes that

- the bore of the connection body for accommodating the bearing bolt of the rotary link and
- the clamping ring

are arranged with an offset, in particular an offset perpendicular to the plane spanned by the Schanz pins. It is possible that the clamping ring and the connection body are part of one single integral connection body.

Another solution of the object of the invention is a pelvic ring injury treatment assembly. The inventive pelvic ring injury treatment assembly comprises a pelvic ring injury treatment device as explained above. Furthermore, the pelvic ring injury treatment assembly comprises two Schanz pins. The Schanz pins are arranged in the Schanz pin accommodations of the pelvic ring injury treatment device.

For one embodiment of the invention, the pelvic ring injury treatment assembly (at least temporarily) comprises a biasing device for applying a biasing force. In this case, the Schanz pins each have a body section designed and configured for being inserted in or driven into the pelvic ring (in particular into the innominate bones), the body section being formed by one end region of the Schanz pin. Furthermore, the Schanz pins each have a biasing section where the Schanz pins can be connected to the biasing device. The pelvic ring injury treatment device is arranged between the body sections and the biasing sections of the Schanz pins. The biasing device is designed and configured for applying a rotation moment about the axes of rotation of the Schanz pin accommodations by applying a biasing force on the biasing sections.

This inventive design of the pelvic ring injury treatment device and of the pelvic ring injury treatment assembly allows a simultaneous compression of both the anterior and posterior joint of the pelvis, enhancing compressibility with a much simpler design than the state of the art, which presumably decreases the time necessary to apply it, is cheaper to produce and offers a more comfortable experience for the patients.

Advantageous developments of the invention result from the claims, the description and the drawings.

The advantages of features and of combinations of a plurality of features mentioned at the beginning of the description only serve as examples and may be used alternatively or cumulatively without the necessity of embodiments according to the invention having to obtain these advantages.

The following applies with respect to the disclosure - not the scope of protection - of the original application and the patent: Further features may be taken from the drawings, in particular from the illustrated designs and the dimensions of a plurality of components with respect to one another as well as from their relative arrangement and their operative connection. The combination of features of different embodiments of the invention or of features of different claims independent of the chosen references of the claims is also possible, and it is motivated herewith. This also relates to features which are illustrated in separate drawings, or which are mentioned when describing them. These features may also be combined with features of different claims. Furthermore, it is possible that further embodiments of the invention do not have the features mentioned in the claims which, however, does not apply to the independent claims of the granted patent.

The number of the features mentioned in the claims and in the description is to be understood to cover this exact number and a greater number than the mentioned number without having to explicitly use the adverb "at least". For example, if an element is mentioned, this is to be understood such that there is exactly one element or there are two elements or more elements. Additional features may be added to these features, or these features may be the only features of the respective product.

The reference signs contained in the claims are not limiting the extent of the matter protected by the claims. Their sole function is to make the claims easier to understand.

BRIEF DESCRIPTION OF THE DRAWINGS

In the following, the invention is further explained and described with respect to preferred exemplary embodiments illustrated in the drawings.

Fig. 1      schematically shows the treatment of a gap of the pelvic ring due to an injury with a pelvic ring injury treatment assembly according to the prior art.

Fig. 2      schematically shows the treatment of a gap of the pelvic ring due to an injury with an inventive pelvic ring injury treatment assembly.

Fig. 3      is a three-dimensional view of a pelvic ring injury treatment assembly comprising a pelvic ring injury treatment device and two Schanz pins.

Fig. 4      shows the pelvic ring injury treatment device of Fig. 3 in a three-dimensional view.

Fig. 5      shows a body part of the pelvic ring injury treatment device of Fig. 4 in an exploded view.

Fig. 6      shows a second bar element of the pelvic ring injury treatment device of Figs. 4 and 5 with a fixing device and a rotary link comprising a connection body in a three-dimensional exploded view.

**Fig. 7**    shows the connection body of Fig. 6 in a three-dimensional view.

**Fig. 8**    shows the connection body of Fig. 7 in a longitudinal sectional view.

**Fig. 9**    shows the connection body of Figs. 7 and 8 in a cross-sectional view.

**Fig. 10**   shows the pelvic ring injury treatment device in a longitudinal sectional view.

## DESCRIPTION OF THE DRAWINGS

In the description and the drawings in some cases components or features being equal or similar to each other have been labelled with the same reference numerals. In these cases, the features or components can be distinguished from each other by an additional letter a, b, ... Preferably the letters a, b distinguish components or features arranged at different ends of the pelvic ring injury treatment device. Reference can be made to these features or components also without the use of the additional letter, in this case one single component or feature being addressed, any number of components or features being addressed or all of the components or features being addressed.

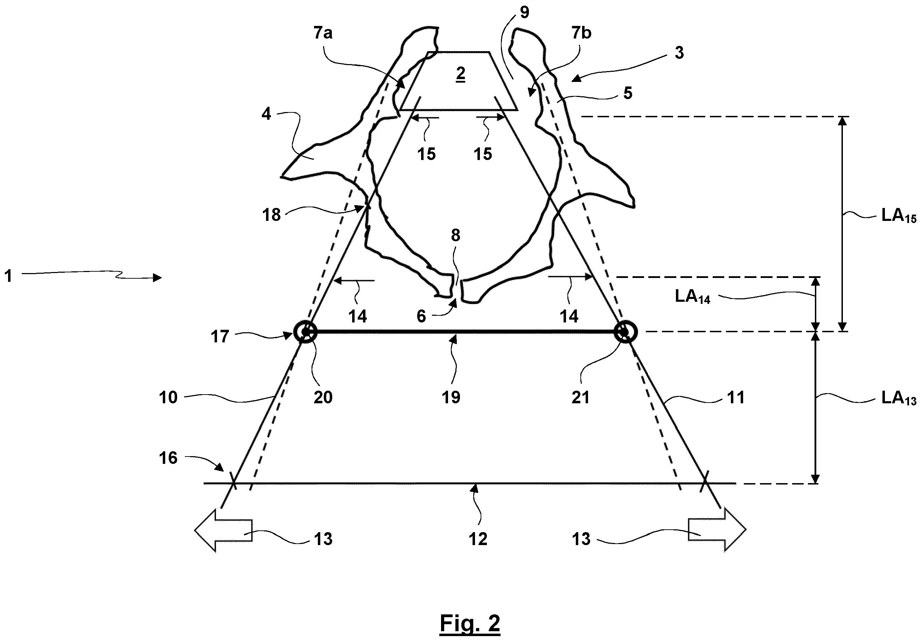

According to **Fig. 2** the Schanz pins 10, 11 have three longitudinal subsections, namely a biasing section 16, an accommodation section 17 and a body section 18. The biasing section 16 serves for interacting with the biasing device 12 such that the biasing force 13 is applied in the biasing section 16 onto the Schanz pins 10, 11. The body section 18 serves for being driven or inserted into the pelvic ring 3, here into the innominate bones 4, 5. The accommodation section 17 serves for providing a connection to a pelvic ring injury treatment device 19. In the accommodation section 17 the pelvic ring injury treatment device 19 is fixed by a fixing device 32 of the pelvic ring injury treatment device 19 in longitudinal direction of the Schanz pins 10, 11 to the Schanz pins 10, 11. However, the Schanz pins 10, 11 can be articulated about an axis of rotation 20, 21 of rotary links 33 of the pelvic ring injury treatment device 19. The axes of rotation 20, 21 have an orientation perpendicular to the drawing plane of Fig. 2 and perpendicular to the plane spanned by the two Schanz pins 10, 11.

The lever arm $LA_{13}$ denotes the effective distance of the connection between the biasing device 12 and the Schanz pins 10, 11 respectively the axial location where the biasing forces 13 bias the Schanz pins 10, 11 from the axial locations where the Schanz pins 10, 11 are connected to the pelvic ring injury treatment device 19. The lever arm $LA_{14}$ denotes the effective distance of the anterior joint forces 14 from the axial point of the Schanz pins 10, 11 where the Schanz pins 10, 11 are connected to the pelvic ring injury treatment device 19. The lever arm $LA_{15}$ denotes the effective distance of the posterior joint forces 15 from the axial point of the Schanz pins 10, 11 where the Schanz pins 10, 11 are connected to the pelvic ring injury treatment device 19. The sum of the moment with respect to the axes of rotation 20, 21 for the Schanz pins 10, 11 leads to the following equation:

$$F_{14} \cdot LA_{14} + F_{15} \cdot LA_{15} = F_{13} \cdot LA_{13}$$

A variation of $LA_{13}$, so a shift of the axes of rotation 20, 21 towards the biasing device 12, leads to a change of the ratio of $LA_{14}$ and $LA_{15}$ which in turn leads to a change of the ratio of the interior joint force 14 and the posterior joint force 15.

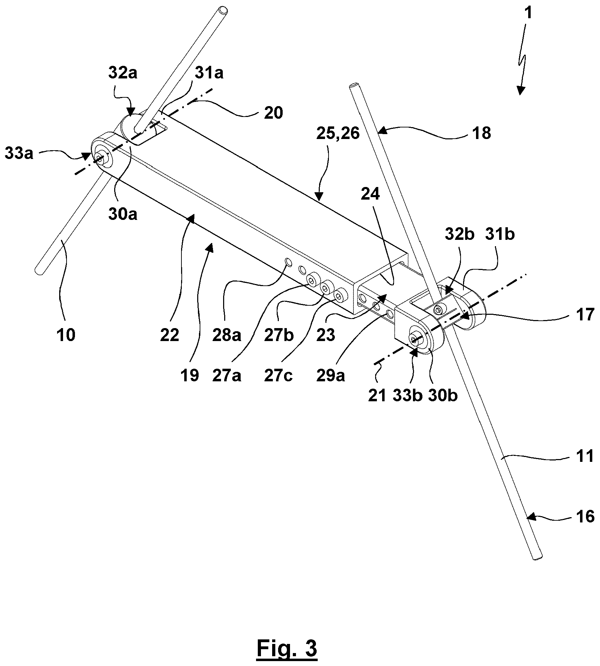

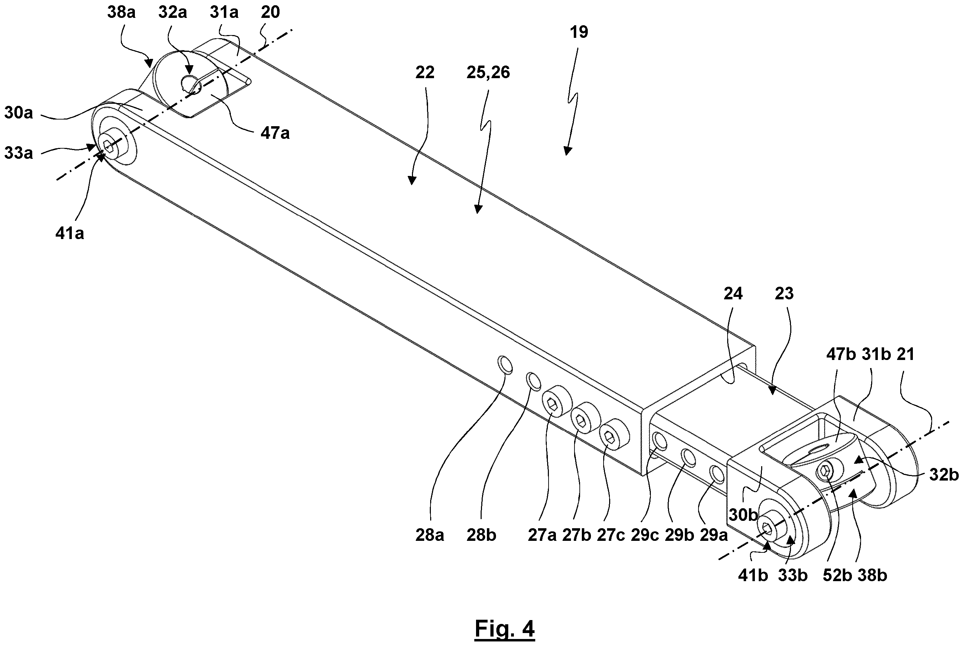

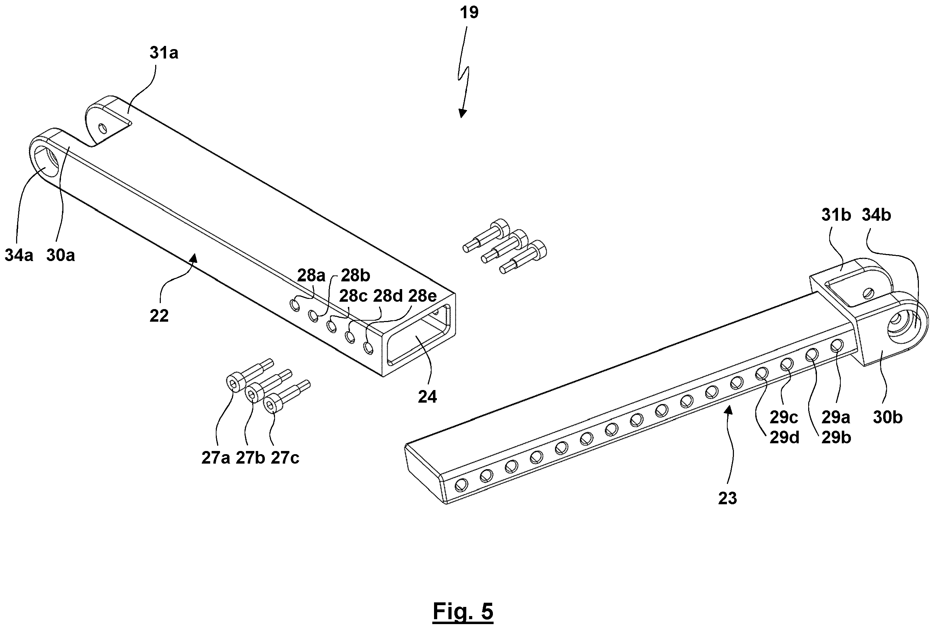

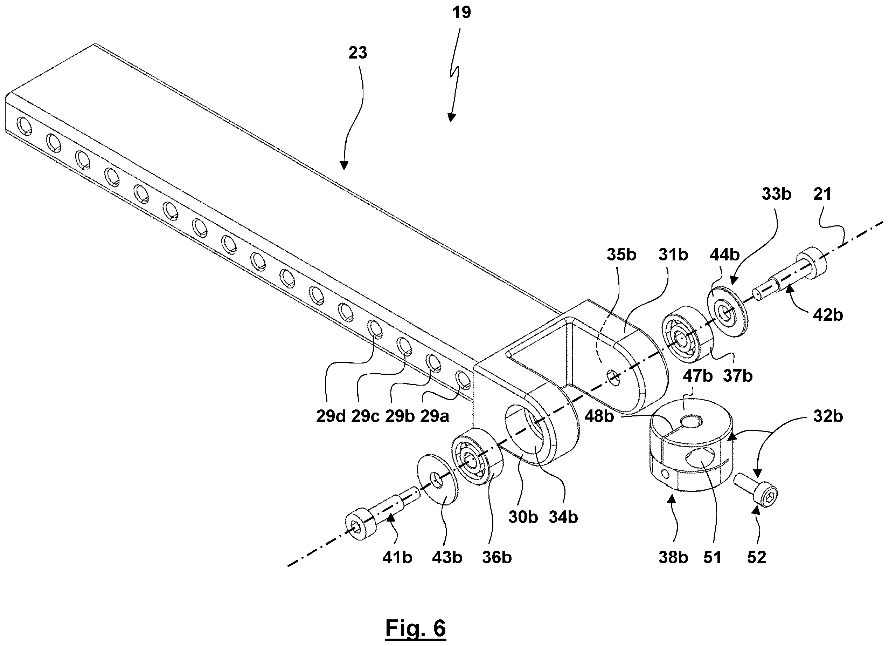

**Fig. 3** shows the pelvic ring injury treatment device 19 with Schanz pins 10, 11 held thereby whereas **Fig. 4** shows the pelvic ring injury treatment device 19, **Fig. 5** shows a base body 25 of the pelvic ring injury treatment device 19 of Fig. 4 and **Fig. 6** shows a bar element 23 of the pelvic ring injury treatment device 19 with the associated fixing device 32 and rotary link 33.

The pelvic ring injury treatment device 19 comprises a first bar element 22 and a second bar element 23. The first bar element 22 comprises a recess 24 wherein the second bar element 23 is accommodated in a sliding fashion. For the shown embodiment the recess 24 and the second bar element 23 have a rectangular cross-section which provides a guidance such that the bar elements 22, 23 cannot be rotated relative to each other about their longitudinal axes.

The first bar element 22 and the second bar element 23 build a base body 25 of the pelvic ring injury treatment device 19 being embodied as a telescopic bar 26. The length of the telescopic bar 26 can be adjusted by an adjustment of the relative position of the bar elements 22, 23.

Once the desired length of the telescopic bar 26 has been achieved, the length of the telescopic bar 26 can be fixed. For the shown embodiment the fixation of the length of the telescopic bar 26 is provided by screws 27 extending through aligned bores 28, 29 of side walls of the bar elements 22, 23. A pair of bores 28, 29 is aligned in specific relative positions of the bar elements 22, 23 such that in a plurality of distant specific positions the screws 27 can be inserted into the bores 28, 29. It is e.g. possible that the bores 29 of the side walls of the second bar element 23 have an inner thread to which the screws 27 are screwed. A plurality of bores 29 is distributed equidistantly along the longitudinal axis of the second bar element 23 whereas at least one bore 28 is arranged in the side wall of the first bar element 22. In the case that more than one bore 28 is arranged in the side wall of the first bar element 22, the bores 28 have the same distances as the bores 29. The use of a plurality of screws 27 as shown improves the fixation of the bar elements 22, 23 to each other.

The free ends of the bar elements 22, 23 form fork-like pairs of bearing walls 30a, 31a respectively 30b, 31b.

In the following the design of a fixing device 32b and a rotary link 33b will be described for the second bar element 23, the fixing device 32b and the rotary link 33b being arranged at the bearing walls 30b, 31b. However, the fixing device 32a and the rotary link 33a arranged at the bearing walls 30a, 31a of the first bar element 23 have the same designs so that the corresponding also applies here.

The bearing walls 30b, 31b comprise bearing lugs 34b, 35b for accommodating bearings 36b, 37b, in particular ball bearings.

The bearings 36, 37 are inserted into the bearing lugs 34, 35 of the bearing walls 30, 31 from the outside in the direction of the axes of rotation 20, 21 and supported in the insertion direction on the bottom of the bearing lugs 34, 35.

A connection body 38 is accommodated between the bearing walls 30, 31. The connection body 38 has a generally cylindrical shape. A cylindrical first subsection of the connection body 38 forms an accommodation body 39 whereas a cylindrical second subsection of the connection body 38 forms a linking body 40.

Bearings bolts 41, 42 (here bearing screws) with discs 43, 44 supported on heads of the bearing bolts 41, 42 are passed through the bearings 36, 37 and connected (here screwed) to (here threaded) bores 45, 46 of the linking body 40. In this way the linking body 40 and so the connecting body 38 including the accommodation body 39 are supported for being rotated about the axes of rotation 20, 21, the axes of rotation 20, 21 being defined by the longitudinal axes of the bearing bolts 41, 42.

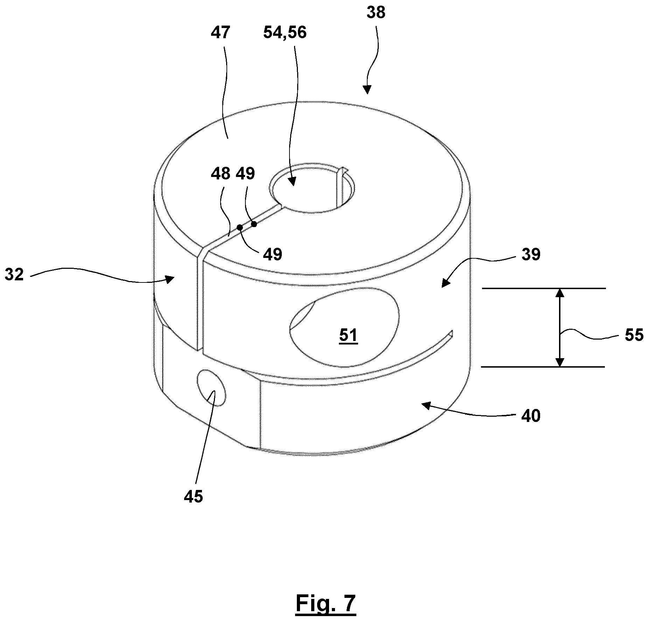

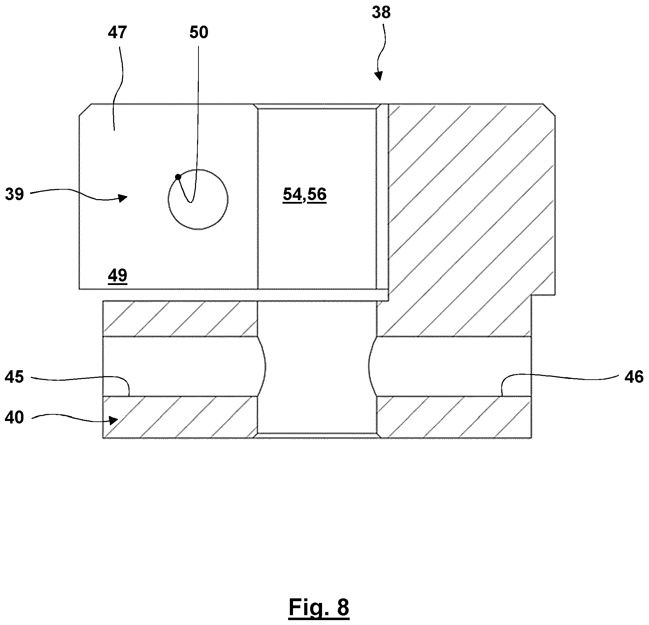

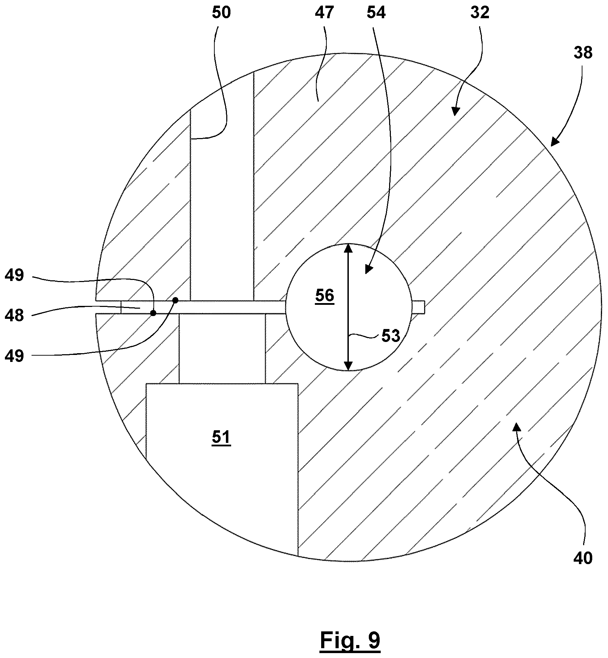

**Figs. 7 and 8** show that the accommodation body 39 is embodied as a clamping ring 47 having a slit 48 defined by the front faces 49 of the clamping ring 47. One end portion of the clamping ring 47 comprises a threaded bore 50 whereas the other end portion of the clamping ring 47 on the other side of the slit 48 comprises an accommodating bore 51 which is aligned to the threaded bore 50. A screw 52 can be inserted into the accommodating bore 51 and the thread of the screw 52 can be screwed with the threaded bore 50. Tightening the screw 52 leads to an elastic bias of the clamping ring 47 with the result that the front faces 49 move towards each other, the slit 48 is at least partially closed and an inner diameter 53 of a bore 56 of the clamping ring 47 reduces (cp. **Fig. 9**). The clamping rings 47 establish Schanz pin accommodations 54.

The fixing devices 32 for axially fixing the Schanz pin accommodations 54 on the Schanz pins 10, 11 are provided by the clamping rings 47.

The rotary link 33 for allowing a pivoting movement of the Schanz pins 10, 11 relative to the base body 25 of the pelvic ring injury treatment device 19 also in the fixed state of the fixing device 32 is provided by the bearing walls 30, 31, the bearing lugs 34, 35, the bearings 36, 37, the bearing bolts 41, 42 and the connection of the bearing bolts 41, 42 to the linking body 40.

The accommodation body 39 and so the fixing position of the fixing device 32 and the connection body 38 and so the axes of rotation 20, 21 of the rotary links 33 have an offset 55. The inner surface of the clamping ring 47 constitutes a bore 56 (having an opening of the cross-section at the slit 48).

One non-limiting option for using the pelvic ring injury treatment assembly 1 comprising the pelvic ring injury treatment device 19 is as follows:
First of all, the Schanz pins 10, 11 are inserted into the pelvic ring 3. In the non-fixing state of the fixing device 32 the pelvic ring injury treatment device 19 is connected to the Schanz pins 10, 11 by inserting the distal ends of the Schanz pins 10, 11 into the bores 56 of the Schanz pin accommodations 54.

The pelvic ring injury treatment device 19 is then slid along the longitudinal axis of the Schanz pins 10, 11 in proximal direction into the desired axial positions.

In the desired axial positions the fixing devices 32 are transferred into the fixing state which means that the clamping ring 47 is closed by tightening the screw 52. In the fixing state of the fixing device 32 the pelvic ring injury treatment device 19 is connected to the Schanz pins 10, 11 at a fixed position but with a remaining degree of freedom for a rotation about the axis of rotation 20, 21 provided by the rotary links 33.

During the sliding movement of the pelvic ring injury treatment device 19 along the Schanz pins 10, 11 the length of the base body 25 can be changed by a relative movement of the bar elements 22, 23. In the end position the length and the relative position of the bar elements 22, 23 is fixed by the screws 27.

Then, the biasing device 12 is attached to the distal ends of the Schanz pins 10, 11 and the biasing forces 13 are applied which lead to the generation of the joint forces 14, 15, a closure of any existing gap 8, 9 and the compression of the innominate bones 4, 5 at the anterior joint 6 and the pressing of the innominate bones 4, 5 against the sacrum 2 at the posterior joints 7a, 7b.

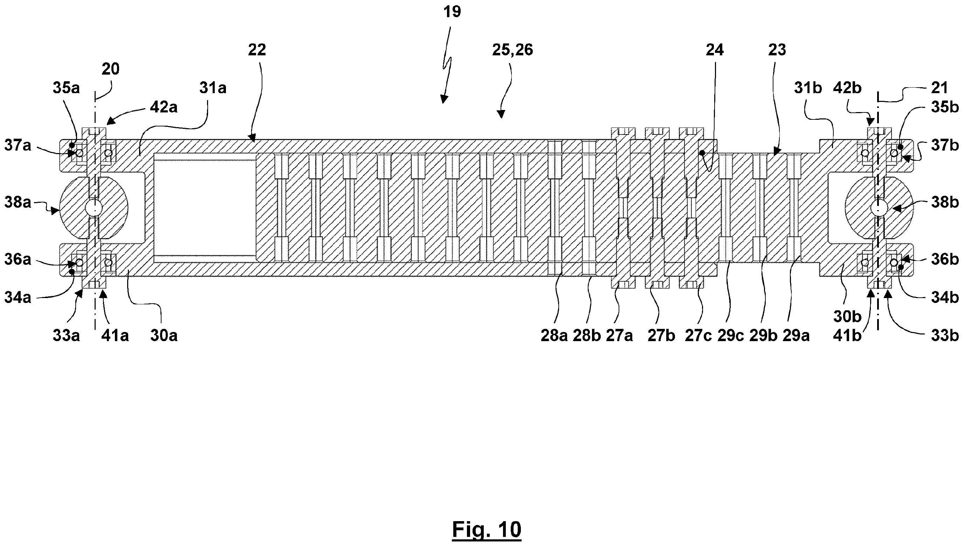

**Fig. 10** shows the pelvic ring injury treatment device 19 in an assembled state without Schanz pins 10, 11 held thereby.

## LIST OF REFERENCE NUMERALS

1    pelvic ring injury treatment assembly
2    sacrum
3    pelvic ring
4    innominate bone
5    innominate bone
6    anterior joint
7    posterior joint
8    gap
9    gap
10   Schanz pin
11   Schanz pin
12   biasing device
13   biasing force

| 14 | anterior joint force |
|---|---|
| 15 | posterior joint force |
| 16 | biasing section |
| 17 | accommodation section |
| 18 | body section |
| 19 | pelvic ring injury treatment device |
| 20 | axis of rotation |
| 21 | axis of rotation |
| 22 | first bar element |
| 23 | second bar element |
| 24 | recess |
| 25 | base body |
| 26 | telescopic bar |
| 27 | screw or bolt |
| 28 | bore |
| 29 | bore |
| 30 | bearing wall |
| 31 | bearing wall |
| 32 | fixing device |
| 33 | rotary link |
| 34 | bearing lug |
| 35 | bearing lug |
| 36 | bearing |
| 37 | bearing |
| 38 | connection body |
| 39 | accommodation body |
| 40 | linking body |
| 41 | bearing bolt |
| 42 | bearing bolt |
| 43 | disc |
| 44 | disc |
| 45 | threaded bore |
| 46 | threaded bore |
| 47 | clamping ring |
| 48 | slit |
| 49 | front face |
| 50 | threaded bore |
| 51 | accommodating bore |
| 52 | screw |
| 53 | inner diameter |
| 54 | Schanz pin accommodation |
| 55 | offset |
| 56 | bore |

**Claims**

1. Pelvic ring injury treatment device (19) comprising

   a) a base body (25) and
   b) two Schanz pin accommodations (54a, 54b) for accommodating two Schanz pins (10, 11), **characterized by**
   c) two fixing devices (32, 32b) for fixing the Schanz pins (10, 11) in the associated Schanz pin accommodations (54a, 54b) and
   d) two rotary links (33a, 33b) for allowing a rotation of the Schanz pin accommodations (54a, 54b) relative to the base body (25) about axes of rotation (20, 21),
   e) wherein the fixing devices (32a,32b) are independent from the rotary links (33a, 33b) such that the Schanz pin accommodations (54a, 54b) can be rotated relative to the base body (25) about the axes of rotation (20, 21) when the fixing devices (32a, 32b) are in the fixing state.

2. Pelvic ring injury treatment device (19) of claim 1, **wherein** the base body (25) has an adjustable length.

3. Pelvic ring injury treatment device (19) of claim 2, **wherein** the base body (25) is a telescopic bar (26).

4. Pelvic ring injury treatment device (19) of claim 3, **wherein** the telescopic bar (26) comprises a first bar element (22) and a second bar element (23), the first bar element (22) comprising a recess (24) wherein the second bar element (23) is arranged for being telescoped.

5. Pelvic ring injury treatment device (19) of claim 4, **wherein** the first bar element (22) and the second bar element (23) comprise transverse bores (28, 29) for accommodating a fixing bolt or fixing screw (27) for fixing one of a plurality of relative positions of the first bar element (22) and the second bar element (23).

6. Pelvic ring injury treatment device (19) of one of the preceding claims, **wherein** at least one end region the base body (25) comprises two parallel bearing walls (30, 31) supporting at least one bearing bolt (41, 42) of the associated rotary link (33).

7. Pelvic ring injury treatment device (19) of claim 6, **wherein** the bearing bolt (41, 42) is supported by a rotational bearing (36, 37) which is arranged in a bearing lug (34, 35) of the bearing wall (30, 31).

8. Pelvic ring injury treatment device (19) of one of the preceding claims, **wherein** at least one Schanz pin accommodation (54) comprises an accommodation body (39) having the shape of a clamping ring (47) comprising a bore (56) and a slit (48) designed and configured for clamping a Schanz pin (10; 11), preferably with a screw (52) connecting the end portions of the clamping ring (47) adjacent to the slit (48) such that screwing the screw (52) to the clamping ring (47) creates and adjusts a clamping force by which the clamping ring (47) clamps the Schanz pin (10; 11).

9. Pelvic ring injury treatment device (19) of one of the preceding claims, **wherein** a connection body (38) comprises a bore (45, 46) for accommodating a or the bearing bolt (41, 42) of the associated rotary link (33), wherein preferably when claim 9 refers back to claim 8

    - the bore (45, 46) for accommodating a or the bearing bolt (41, 42) and
    - the clamping ring (47)

are arranged with an offset (55).

10. Pelvic ring injury treatment assembly (1) comprising a pelvic ring injury treatment device (19) of one of the preceding claims and two Schanz pins (10, 11) arranged in the Schanz pin accommodations (54) of the pelvic ring injury treatment device (19).

11. Pelvic ring injury treatment assembly (1) of claim 10, **wherein**

    a) the Schanz pins (10, 11) each have a body section (18) and a biasing section (16), the pelvic ring injury treatment device (19) being arranged between the body sections (18) and the biasing sections (16), and
    b) a biasing device (12) is connected to the biasing sections (16), the biasing device (12) being designed and configured for applying a rotation moment about the axes of rotation (21, 22) of the rotary links (33) by applying a biasing force on the biasing sections (16).

EP 4 656 147 A1

**Fig. 1**
(PRIOR ART)

**Fig. 2**

EP 4 656 147 A1

Fig. 3

Fig. 4

**Fig. 5**

Fig. 6

EP 4 656 147 A1

47  54,56  38

48 49

54,56

32

49

39

51

55

45

40

**Fig. 7**

**Fig. 8**

Fig. 9

Fig. 10

Europäisches
Patentamt
European
Patent Office
Office européen
des brevets

# EUROPEAN SEARCH REPORT

**Application Number**

EP 24 17 8411

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/236171 A1 (CHAN DANIEL [US]) 5 August 2021 (2021-08-05) | 1-3,9-11 | INV. A61B17/64 A61B17/66 |
| Y | * paragraphs [0048] - [0054], [0061]; figures 1,2 * | 8 | |
| X | US 4 361 144 A (SLAETIS PAER E V ET AL) 30 November 1982 (1982-11-30) | 1-4,9-11 | |
| Y | * column 2, lines 15-60; figures 1-3 * | 6,7 | |
| X | DE 195 16 310 A1 (MAN CERAMICS GMBH [DE]) 7 November 1996 (1996-11-07) | 1-4,9-11 | |
| Y | * column 5, line 26 - column 6, line 52; figures 3,4 * | 5 | |
| Y | US 8 398 637 B2 (PARSELL DOUGLAS ERIC [US]; COLE PETER ALEXANDER [US]) 19 March 2013 (2013-03-19) * column 4, lines 15-44; figures 4-8 * | 5 | |
| Y | WO 90/11727 A1 (HARDY JEAN MARIE [FR]) 18 October 1990 (1990-10-18) * page 7, line 28 - page 9, line 12; figures 1-3 * | 6,7 | **TECHNICAL FIELDS SEARCHED (IPC)** A61B |
| Y | US 2003/187432 A1 (JOHNSON TAB C [US] ET AL) 2 October 2003 (2003-10-02) * paragraph [0039]; figures 1,2 * | 8 | |

The present search report has been drawn up for all claims

| Place of search | Date of completion of the search | Examiner |
|---|---|---|
| Munich | 11 November 2024 | Fourcade, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 8411

This annex lists the patent family members relating to the patent documents cited in the above-mentioned European search report.
The members are as contained in the European Patent Office EDP file on
The European Patent Office is in no way liable for these particulars which are merely given for the purpose of information.

11-11-2024

| Patent document cited in search report | | Publication date | Patent family member(s) | | Publication date |
|---|---|---|---|---|---|
| US 2021236171 | A1 | 05-08-2021 | US | 2021236171 A1 | 05-08-2021 |
| | | | WO | 2019236630 A1 | 12-12-2019 |
| US 4361144 | A | 30-11-1982 | NONE | | |
| DE 19516310 | A1 | 07-11-1996 | NONE | | |
| US 8398637 | B2 | 19-03-2013 | NONE | | |
| WO 9011727 | A1 | 18-10-1990 | CA | 2031302 A1 | 12-10-1990 |
| | | | EP | 0423299 A1 | 24-04-1991 |
| | | | FR | 2645428 A1 | 12-10-1990 |
| | | | JP | H03505295 A | 21-11-1991 |
| | | | WO | 9011727 A1 | 18-10-1990 |
| US 2003187432 | A1 | 02-10-2003 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 111529029 A
- EP 2837346 A1
- DE 102010032465 A1

**Non-patent literature cited in the description**

- **A. QUEIPO-DE-LLANO** ; **A. PEREZ-BLANCA** ; **F. EZQUERRO** ; **F. LUNA-GONZÁLEZ**. Simultaneous anterior and posterior compression of the pelvic ring with external fixation using a pre-tensed curved bar: A biomechanical study. *Injury, Int. J. Care Injured*, 2013, vol. 44, 1787-1792